# EUROPEAN PATENT APPLICATION

(11) **EP 2 147 663 A1**
(43) Date of publication of application: **27.01.2010**
(21) Application number: 08013292.1
(22) Date of filing: 23.07.2008
(51) Int. Cl.: A61F 2/84

(54) **Stent movement preventing device**

(71) Applicant: Abbott Laboratories Vascular Enterprises Limited, Terrace, Dublin 2 (IE)
(72) Inventor: Jeffrey, Andrew, 72074 Tübingen (DE)
(74) Representative: Schmitz, Hans-Werner

(57) **Abstract**

A device for preventing stent movement during delivery comprising a securement connector arranged to engage a catheter, at least one flexible connecting member, said flexible connecting member having a first end portion and a second end portion, the first end portion coupled to said securement connector, and a locking member located at said flexible connecting member second end portion arranged to engage a portion of a stent comprising struts defining gaps therebetween wherein said locking member takes the form of a clasp section snapping into one gap of said gaps between the struts.

## Description

The present invention relates to a device for preventing stent movement during delivery into a lumen of a body vessel and a stent delivery system.

A mechanism to improve stent securement is known from US 2004/0236406 A1. This device comprises a securement connector arranged to engage a catheter, at least a flexible connecting member coupled to the securement connector and a locking member arranged to engage a portion of a stent. For the engagement of the locking member with the stent, however, an associated engagable portion formed on at least one end of the stent has to be provided. Thus said device is only usable with a modified type of stent.

It is, therefore, an object of the present invention to provide a device for preventing stent movement that can be used without additional engagable portions on the stent to be provided, and, therefore, simplifies the overall connecting structure to prevent a stent movement.

The solution of this object is achieved by the features of claim 1 and 10, respectively. A device for preventing stent movement according to the present invention comprises a securement connector arranged to engage a catheter and at least one flexible connecting member. Said flexible connecting member has a first portion and a second portion, the first portion being coupled to said securement connector. Further said device for preventing stent movement comprises a locking member located at said flexible connecting member second end portion. This locking member is arranged to engage a portion of a stent comprising struts defining gaps therebetween wherein said locking member takes the form of a clasp section snapping into one gap of said gaps between the struts.

As a further aspect of the invention a stent delivery system is disclosed comprising a catheter, an expandable balloon and a radially expandable stent having at least one engagable portion which is characterized by a device for preventing stent movement according to the present invention, which comprises a securement connector arranged to engage a catheter and at least one flexible connecting member. Said flexible connecting member has a first portion and a second portion, the first portion being coupled to said securement connector. Further said device for preventing stent movement comprises a locking member located at said flexible connecting member second end portion. This locking member is arranged to engage a portion of a stent comprising struts defining gaps therebetween wherein said locking member takes the form of a clasp section snapping into one gap of said gaps between the struts. So, as a specific advantage, a standard type stent without any modifications can be secured by simply using the gaps of the stent structure as the technical feature cooperating with the clasp section of the locking member.

The dependent claims contain advantageous embodiments of the present invention.

A method of assembling a device for preventing movement of a stent is defined in claim 12 comprising the following method steps: mounting the stent in a pre-crimped state over the balloon working length of a balloon; positioning the stent over clasp sections of locking members of the device for preventing stent movement during delivery ensuring that the clasp sections are aligned between the struts of the stent; inserting the entire assembly of the stent and the preventing device into a crimp head of a crimping device crimped in the entire assembly to its nominal diameter.

Further features and advantages of the present invention will become apparent from the following description of preferred embodiments with reference to the appended drawings, wherein
Fig. 1 shows an enlarged perspective view of the device for preventing stent movement according to the present invention,
Fig. 2 shows an enlarged cross sectional view of a distal part of a catheter comprising the device for preventing stent movement according to the present invention in an inflated state of the balloon,
Fig. 3 shows an enlarged cross sectional view of a stent delivery system according to the present invention in a deflated state of the balloon, and
Fig. 4 shows an enlarged perspective view of a further embodiment of the device for preventing stent movement according to the present invention.

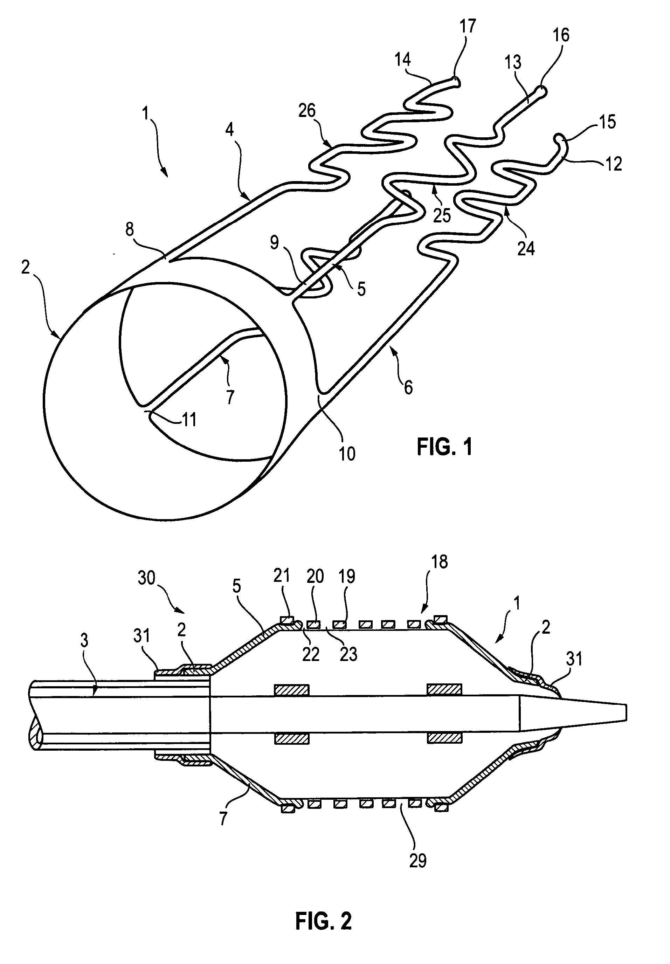

In the perspective view of Fig. 1 the device 1 for preventing stent movement is depicted. The device 1 comprises on its proximal end a securement connector 2 in the form of a ring to be connected with a catheter 3. A plurality of flexible connecting members 4, 5, 6, 7 are connected on respective first end portions 8, 9, 10, 11 with said securement connector 2 and extend in a distal direction via serpentine bands 24, 25, 26 towards respective second end portions 12, 13, 14, of the device 1. The second end portions 12, 13, 14, are formed with locking members 15, 16, 17 for the engagement with a portion of a stent 18 not shown in Fig. 1.

Fig. 2 shows an enlarged cross sectional view of a distal part of a catheter comprising the device for preventing stent movement according to the present invention in an inflated state of a balloon 29. As can be seen from Fig. 2, both a first securement connector 2 at the proximal end of the balloon 29 and a second securement connector 2 at the distal end of the balloon are employed to fix the stent 18 each using connecting members 4 - 7. Due to this cross sectional view only the connecting members 5, 7 are visible whilst the other connecting members 4, 8 are not visible. The securement connector 2 of each device 1 is connected with the catheter 3 via a shrink tube 31, respectively. The connecting members 4 - 7 are flexible and expand with the balloon 29 via their serpentine bands 24 - 26 as illustrated in the inflated state of the balloon 29 in Fig. 2. The locking members 15, 16, 17 formed at the second end portions 8 - 11 of the connecting members 4 - 7 respectively, comprise the form of a clasp section snapping, as shown in Fig. 2 e. g. into one gap 22 of gaps 22, 23 between two adjacent struts 20, 21 of said struts 19 - 21 of the stent 18 being identified by reference signs representative for all struts and gaps of the stent. Thus, without the need of modifications of the stent structure the stent 18 is fixed from the proximal end as well as the distal end via the device1, respectively and reliably prevented from moving.

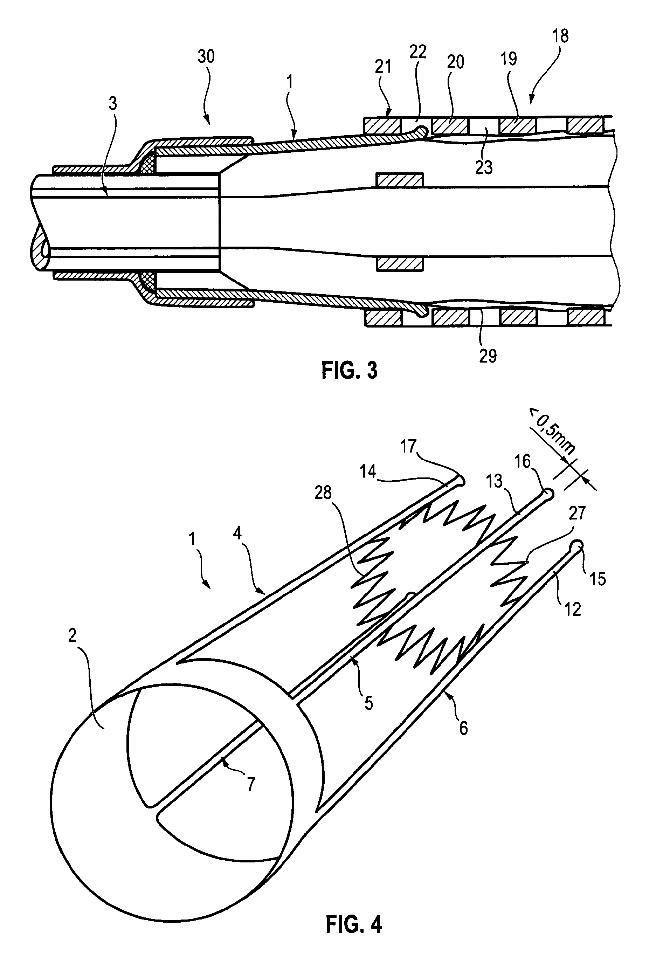

Fig. 3 shows an enlarged cross sectional view of stent delivery system 30 according to the present invention comprising the catheter 3, the expandable balloon 29 and the radially expandable stent 18 in a deflated state of the balloon 29. When the balloon 29 is deflated the flexible connecting members 4 - 7 which are formed from a shape memory material (or other flexible material like nitinol or elastic polymer) bend back and retain their generally linear shape, as shown in Fig. 1, wherein the locking members 15 - 17 on their second end portions 12 - 14 remaining engaged within respective gaps 22, 23 between the struts 19 - 21 of the stent 18. In Fig. 3 the same connecting configuration as in Fig. 1 is exemplarily depicted with the two connecting members 5, 7 snapping into the gap 22 between the struts 20, 21 of the stent 18. However, upon retraction of catheter 3 the locking members 15 - 17 easily slide out of the respective gaps 22 in order to release stent 18.

Fig. 4 shows an enlarged perspective view of a further embodiment of the device for preventing stent movement according to the present invention. In this embodiment highly flexible prongs 27, 28 are provided to connect the connecting members 4 - 7 in a region adjacent to their second end portion 12 - 14 for a further improved fixation of a non-modified stent 18.

In addition to the written disclosure reference is herewith made explicitly to the disclosure of the invention in Fig. 1 to 4.

### List of reference signs

- 1: device for preventing stent movement
- 2: securement connector
- 3: catheter
- 4,5,6,7: connecting member
- 8,9,10,11: first end portion
- 12,13,14: second end portion
- 15,16,17: locking member
- 18: stent
- 19,20,21: struts
- 22,23: gaps, engagable portions
- 24,25,26: serpentine band
- 27,28: prongs
- 29: balloon
- 30: stent delivery system
- 31: shrink tube

## Claims

1. A device (1) for preventing stent movement during delivery comprising:
- a securement connector (2) arranged to engage a catheter (3) ;
- at least one flexible connecting member (4 - 7), said flexible connecting member (4 - 7) having a first end portion (8 - 11) and a second end portion (12 - 14), the first end portion (8 - 11) coupled to said securement connector (2), and
- a locking member (15 - 17) located at said flexible connecting member second end portion (12 - 14) arranged to engage a portion of a stent (18) comprising struts (19 - 21) defining gaps (22, 23) therebetween;
wherein said locking member (15 - 17) takes the form of a clasp section snapping into one gap (22) of said gaps (22, 23) between the struts (19 - 21).

2. The device of claim 1 comprising a plurality of flexible connecting members (4 - 7).

3. The device of claim 1 or 2 wherein said flexible connecting member (4 - 7) is adapted to expand with the stent (18).

4. The device of one of claims 1 to 3, wherein each connecting member (4 - 7) comprises a serpentine band (24, 25, 26) being disposed between the first end portion (8 - 11) and the locking member (15 - 17).

5. The device of one of claims 1 to 4, wherein the flexible connecting member (4 - 7) is balloon expandable.

6. The device of one of claims 1 to 5, wherein the flexible connecting member (4 - 7) is made from a shape memory material.

7. The device of claim 6, wherein the shape memory material has an Af temperature lower than the normal human body temperature.

8. The device according to one of claims 1 to 7, wherein a shrink tube (31) connects the securement connector (2) with the stent (18)

9. The device according to one of claims 1 to 8, wherein prongs (27, 28) connect the connecting members (4 - 7) in a region adjacent to their second end portion (12 - 14)

10. A stent delivery system (30) comprising:
a catheter (3);
an expandable balloon (29), and
a radially expandable stent (18) having at least one engagable portion (22) **characterized by** a device (1) according to one of claims 1 - 8.

11. The stent delivery system of claim 10 wherein said device (1) and said stent engagable portion (22) are adapted to remain engaged until said balloon (29) is deflated.

12. Method of assembling a device (1) for preventing movement of a stent during stent delivery, said stent including a stent body having struts (19 - 21) comprising:
mounting the stent (18) in a pre-crimped state over the balloon working length of a balloon (29);
positioning the stent (18) over clasp sections of the locking members (15, 16, 17) of the device (1) for preventing stent movement during delivery ensuring that the clasp sections are aligned between the struts (19 - 21) of the stent (18);
inserting the entire assembly of the stent (18) and the preventing device (1) into a crimp head of a crimping device in which the entire assembly is crimped to its nominal diameter.
